# EUROPEAN PATENT APPLICATION

(11) **EP 3 992 986 A1**
(43) Date of publication of application: **04.05.2022**
(21) Application number: 20204740.3
(22) Date of filing: 29.10.2020
(51) Int. Cl.: G16H 50/20

(54) **CLOUD-BASED DIAGNOSTIC SYSTEM FOR PROVIDING AN ASSESSMENT OF A HEALTH STATUS**

(71) Applicant: Babelli, Ibrahim Mahmoud M., 12232 - 7582 Riyadh (SA)
(72) Inventor: Babelli, Ibrahim Mahmoud M., 12232 - 7582 Riyadh (SA)
(74) Representative: LifeTech IP Spies & Behrndt Patentanwälte PartG mbB

(57) **Abstract**

Disclosed is a cloud-based diagnostic system (100) for providing an assessment of a health status of an animal (10) or human (13, 15) to a user (60). The assessment is based on input data associated with the health status of the animal (10) or human (13, 15), and on a confirmation (70, 71, 72, 73) of one or more medical experts (50, 51, 52). The diagnostic system (100) comprises an interface (110), configured to collect the input data, and a data processing unit (120), configured to perform a preliminary diagnosis, based on the input data, of a potential illness of the animal (10) or human (13, 15), to provide the preliminary diagnosis to the one or more medical experts (50, 51, 52) in order to obtain the confirmation (70, 71, 72, 73), and to provide a result (140, 141, 142, 143) of the confirmation (70, 71, 72, 73) to the user (60).

## Description

The present invention relates to a cloud-based diagnostic system and method for providing an assessment of a health status of an animal or human, and in particular to a system and method to identify animal and human illness.

### BACKGROUND

The state of the art knows systems configured to identify particular aspects of a health status of an animal or a human. For example, measuring heart and breathing rates in zoo animals from a distance can be achieved by contactless monitoring of cardiopulmonary signals, inferred from high-resolution video images, with the prospect of easing the challenge for veterinarians particularly in conditions when contact with a conscious animal is inconvenient, difficult, or distressing to the animal.

Video imaging also allows a facial recognition of animals. For example, the document US10643062 discloses a system for facial recognition and identification of a pet. In another commercial application, imaging technology is employed to identify individual cows by their features. Furthermore, at least experimentally, facial recognition has been used to detect expressions of pain in sheep. In order to achieve this task, artificial intelligence systems were developed which use a couple of different facial expressions to recognize whether a sheep is in pain, and to estimate the severity of that pain. The results may be applied to other types of animals, such as rabbits or horses.

A variety of audio detectors are used in the state of the art to detect e.g. a breathing sound of animals. For example, US6952912B2 describes a device in which a transducer or microphone is placed in direct contact with the skin of the animal. The document also describes a method for recording respiratory sounds in exercising horses for an identification of upper airway abnormalities, and for evaluating athletic potential.

The document US10041843 describes a system configured to measure the core temperature of a warm-blooded animal by means of a skin temperature sensor. The measurement is corrected using measurements from an ambient temperature sensor and an accelerometer to sense the movement of the animal.

Sensors for animal odor detection using artificial 'noses' have become widespread and reliable. For example, US9279791B2 discloses an odor sensing system comprising chromatographic membranes, a plurality of sensors, and a pattern analyzer configured to identify the odor molecules. Mimicking biological olfaction, electronic 'noses' are employed to detect a variety of important compounds in complex environments, serving as a source of capturing elements for biosensing.

In the state of the art, sensor equipment as described here has been integrated into systems which are specifically adapted to monitor vital signs and health status of an animal or human.

In particular, artificial intelligence has been employed to help veterinarians perform diagnoses of animals. For example, a commercially available application for mobile devices supports dairy farmers in diagnosing sick cows in a herd. The application, using machine learning algorithms, asks the farmers a series of questions, and combines the response with regional farm data and weather data to produce a list of potential causes of disease in the animals. A further commercially available artificially intelligent medical search tool is available for veterinarians, configured to provide access to a medical online library, and render animal health diagnostics both easier and more accurate.

In the patent literature, WO2020010694A1 discloses a system configured to estimate, again by means of an artificial intelligence, a morbidity of an animal from video and in particular from sound data. The estimate is based on classifying picture frame and sound frame features, as well as an activity of the animal.

Patent US10628756B1 discloses a system which classifies a health status of an animal based on tracing locations of the animal.

US20200065966 describes a system and methods for detecting udder diseases in cattle, using image processing techniques coupled with temperature measurement and machine-learning supported data processing.

While the cited references allow a monitoring of a health status of an animal or support a veterinarian in his work, providing a diagnosis is still achieved through direct physical examination by a veterinarian. However, taking the animal to the veterinarian, or bringing the veterinarian to the animal, has several shortcomings, especially when a dense net of veterinarian assistance is unavailable. In situations where animals requiring veterinary care are distributed over a large area with limited access, such as camels in the desert, or cattle in remote farms, or if the owner of the animal cannot afford conventional veterinary care for the animals, then this conventional method fails to provide the required care timely and inexpensively.

A case in point is the cattle industry in Ethiopia. There, as in many other East African countries, the majority of cattle owners are organized as family farms with small numbers of animals, and access to veterinary care is far and cost-intensive.

These considerations, as well as the references cited above, show that there is a demand for automated diagnostic systems for animals in situations where seeing a veterinarian cannot be easily achieved. A demand also exists for humans in such situations, in particular if conversation is impeded or impossible, as would be the case for infants, or for disabled patients.

### SUMMARY OF THE INVENTION

At least some of the problems addressed above are overcome by a diagnostic system for assessing a health status of an animal or human according to claim 1, a mobile device according to claim 9, a portable and attachable device according to claim 10, a method for assessing a health status of an animal or human according to claim 11, and a computer program product according to claim 12. The dependent claims refer to further advantageous realizations of the subject matter of the independent claims.

The present invention relates to a cloud-based diagnostic system for providing an assessment of a health status of an animal or human to a user. The assessment is based on input data associated with a health status of the animal or human, and on a confirmation of one or more medical experts (e. g., veterinarians), which are advantageously located remotely both from the user and from the animal or human. The diagnostic system comprises an interface which is configured to collect the input data. The diagnostic system further comprises a data processing unit, which is advantageously realized within a data center for access via the internet as a cloud computing system, providing both storage and computing power. The data processing unit is configured to perform a preliminary diagnosis of a potential illness of the animal or human, based on the input data obtained via the interface. The data processing unit is further configured to provide the preliminary diagnosis to one or more medical experts in order to obtain the confirmation. The data processing unit is further configured to provide a result of the confirmation to the user.

The animal maybe domesticated, as e. g. any kind of livestock such as a cow, a sheep, a horse, a pig or a goat, or any kind of pet such as a dog, a cat, or a bird. The animal may also be e. g. a race horse, or a police dog.

The term "user" refers to a person interested in obtaining a diagnosis for the animal or human by means of the diagnostic system. The user may e.g. be an owner, keeper, herdsman, or caretaker of the animal, or a person related to the human.

The input data is advantageously generated by a variety of sensors. These sensors are located in proximity to the animal or human, and configured to sense environmental conditions pertaining to the health status of the animal or human. The collection may comprise a wireless communication of the input data. In particular, the interface comprises a means to convey the input data to the data processing unit, or respectively to the cloud computing environment which hosts the data processing unit. Advantageously, the interface is configured to employ a wireless transmission of data to the data processing unit, which may run through an existing cellular network or over a wireless wide area network (WWAN).

The data processing unit may be configured to perform the preliminary diagnosis by means of adaptive learning or machine learning. The data processing unit may in particular be programmed with an artificial intelligence based on an artificial neural network, adapted to improve the preliminary diagnosis by optimizing a correlation between input data, the preliminary diagnoses, confirmations, and results. Here, a basis for machine learning is provided in particular by the confirmations of the medical experts, i. e. the data processing unit improves the way in which it performs the preliminary diagnosis in particular by learning from feedback from the medical experts. If the diagnostic system provides preliminary diagnoses for more than one animal of a same kind and/or within a proximity (e.g. using a same shed, belonging to a same owner (or user), or living in a same geographical region or neighborhood), the data processing unit may be configured to include, in the learning process, also data from different such animals. In particular, the data processing unit may be configured to compare a present or past health status of one animal with a present or past health status of another animal, along with according preliminary diagnoses, confirmations, and results.

The data processing unit may comprise an electronic platform, which is advantageously located remotely from both the user and the animal or human, and which is configured to perform data storage and computing tasks and to provide an access for the one or more medical experts. Together with the data processing unit, the electronic platform may in particular also be part of a cloud computing environment. The access for medical experts is advantageously realized by a data connection for exchanging data via the internet, e.g. by means of a computer or mobile device of the medical expert. The medical experts can be located anywhere around the world. According to embodiments of the proposed diagnostic system, the data processing unit provides accounts for medical experts who wish to participate in confirming the preliminary diagnoses generated by the data processing unit.

The data processing unit may be configured to automatically create an account upon request from a medical expert, and to include, in the creation process, a review of credentials of the medical expert. The data processing unit is advantageously configured to notify the one or more medical experts if a preliminary diagnosis has been performed. The processing unit may in particular be configured to discriminate preliminary diagnoses which suggest that the animal or human is healthy from preliminary diagnoses which suggest that the animal or human suffers or potentially suffers from an illness. According to embodiments, medical experts are only informed in the latter case, i. e. if the preliminary diagnosis suggests that that the animal or human (potentially) suffers from an illness.

In an embodiment of the diagnostic system, the data processing unit is configured to send the preliminary report e.g. by email to the one or more medical experts. In a further embodiment of the diagnostic system, the processing unit is configured to store the preliminary diagnosis, to provide information about its existence to the one or more medical experts, and to grant access to any of them upon request. In particular, the data processing unit may be configured to request the one or more medical experts to log into the cloud computing environment. The data processing unit may be configured to record an activity of a medical expert in order to attribute a financial compensation based on the confirmations by the medical expert.

The data processing unit may be further configured to notify the user about the diagnosis. According to embodiments, this notification may occur by email or text message. The data processing unit may be configured to notify the user if a preliminary diagnosis has been reviewed by at least one of the one or more medical experts, providing a result of the confirmation. The result may e.g. comprise an endorsement of the preliminary diagnosis, a rejection of the preliminary diagnosis, or ambiguous. The notification may comprise further instructions by the one or more medical experts. The notification may also comprise further instructions added by the data processing unit. Notifications may be sent to the user, or prepared for retrieval by the user, every time the data processing unit performs a preliminary diagnosis, or just in cases where the preliminary diagnosis suggests a potential illness of the animal or human.

Optionally, the processing module is configured to process, as input data, sensor data from one or more of the following telemetric sensors: a visual camera, a temperature sensor, an audio detector, and/or an odor sensor. The adjective "telemetric" here refers to sensors without a direct contact to (i.e., not touching) the animal or human. These sensors may be mounted e. g. in a shed of an animal, in a cradle of an infant, or on a frame of a bed of an adult human patient. In embodiments, the temperature sensor is a thermometer or an infrared sensor. The audio detector may be a microphone positioned to detect e.g. breathing, utterings, and/or sounds of movements of the animal. The odor sensor may in particular be adapted for an analyzation of odors from feces and/or sweat.

Optionally, the processing unit is further configured to process, as input data, sensor data of one or more of the following contact-based sensors: a sensor for detecting body temperature, a heartbeat or pulse sensor, a sensor for detecting respiratory sounds, and/or a sensor for measuring blood pressure, like e. g. a sphygmomanometer. Here, the term "contact-based" refers to a direct contact with the animal or human. For some embodiments of the diagnostic system, contact-based sensors may be added to telemetric sensors as described above, but other embodiments of the diagnostic system may be configured to operate with input data derived only from contact-based sensors.

Optionally, the data processing unit is configured to keep a record of the input data, and to provide the preliminary diagnosis based on one or more of the following: benchmarks or thresholds derived from input data, predefined benchmarks or thresholds to be compared to the input data, a rate of change of the input data, and/or an automatic recognition of facial features of the animal or human. Thus the preliminary diagnosis may be automatically based on anamnestic data, i. e. the processing module may be configured to compare input data associated with an animal to previous input data, preliminary diagnoses, and/or confirmations of the same animal or human. This is in particular beneficial to distinguish changes in an expression, movement or habit, temperature, sweat, odor or other medically relevant features which are outside of benchmarks for, or exceed minimum and maximum bounds of, typical long-term values of these data for this particular animal or human.

The preliminary diagnosis may also be based on predefined benchmarks or thresholds, wherein the term "predefined" refers to available typical values of these quantities for the type of animal, or for humans in general, rather than for the individual. The diagnosis may also be based on a rapidity of a change of these data. Changes may be in particular recorded in the patterns of an animal facial profile, e. g. by comparing a standard reference set of images of the same animal, recognizing features such as contortions, a change in the eye (like e.g. a color of a sclera), and/or a color of the face or tongue of the animal.

Similarly to the description above, the preliminary diagnosis may also be based on benchmarks or thresholds derived from another animal or human for which the diagnostic system has provided a preliminary diagnosis.

Optionally, the diagnostic system further comprises a medical diagnosis database for storing data adapted to identify a potential illness. Furthermore, the data processing unit includes an artificial intelligence module and is configured to receive the data from the medical diagnosis database, and to perform the preliminary diagnosis based on the data from the medical diagnosis database, and with support from the artificial intelligence module.

The medical diagnosis database may comprise data adapted to perform the diagnosis on various levels. The data may e. g. be suited for a diagnosis by pattern recognition, or for a differential diagnosis. The data processing unit may be adapted to perform, based on the input data and on data from the diagnosis database, a differential diagnosis. The medical diagnosis database may further be (or be part of) a clinical decision support system (CDSS). In these cases, the data processing unit may be configured to operate the CDSS in order to perform the preliminary diagnosis.

Optionally, the data processing unit is configured to keep a record of preliminary diagnoses, confirmations, and/or results, and to carry out, by means of the artificial intelligence module, a learning algorithm, and to perform the preliminary diagnosis based on the record and on the learning algorithm.

Similarly to the description above, the processing unit may be configured to perform the preliminary diagnosis by means of adaptive learning or machine learning, carried out in the artificial intelligence module, and adapted in particular to include both the anamnestic data as well as the data from the medical diagnosis database. The artificial intelligence module may in particular be programmed with an artificial intelligence based on an artificial neural network, adapted to improve the preliminary diagnosis by optimizing a correlation between input data, stored preliminary diagnoses, confirmations, and results, and data from the medical diagnosis database.

Optionally, the data processing unit is configured to establish a communication channel between the user and at least one of the one or more medical experts. The communication channel may be established by providing contact details. However, in further embodiments, the communication channel is provided as a text message, as an online chat, and/or as a voice and/or a video connection. The communication channel may in particular run through the interface and/or the data processing unit.

Optionally, the processing unit is configured to process, as input data, output data from a mobile test device for providing test results associated with body fluids such as blood, urine, saliva or body excrements such as dung or vomit. In particular, mobile blood test devices known in the state of the art are adapted to perform a complete blood count (CBC) analysis. The mobile test device may also be adapted to perform a serum test, a chemistry or thyroid panel, a nutrient test, and/or a test for enzyme markers. The mobile test device may yield input data pertaining e.g. to cholesterol, blood sugar, or glucose levels, or to tests for liver or kidney function.

Embodiments further refer to a mobile device, configured to connect to the diagnostic system, and to receive the result of the confirmation. The mobile device may in particular be a smartphone, tablet, or notebook. The mobile device may be configured, by means of a software application, to communicate with the data processing unit, or respectively connect to the cloud computing environment. In particular, if a communication channel is established between the user and one of the one or more medical experts, the communication channel may go through the mobile device. According to some embodiments, the mobile device may be further configured to relay data from sensors as input data to the interface. The mobile device may comprise the interface, or a part of the interface.

Embodiments further refer to a portable device which is adapted to be attachable to the animal or human. The portable device comprises one or more sensors configured to produce sensor data, and may in particular comprise contact-based sensors of the kind described above. The portable device further comprises a data connection configured to convey the sensor data to the diagnostic system. The attachable device may comprise a collar, a harness, and/or an inflatable cuff.

The present invention furthermore relates to a method for providing an assessment, based on input data associated with a health status of the animal or human and on a confirmation of one or more medical experts, of a health status of an animal or human to a user. The method comprises the steps:
collecting the input data;
performing a preliminary diagnosis, based on the input data, of a potential illness of the animal or human,
providing the preliminary diagnosis to one or more medical experts in order to obtain the confirmation; and
providing a result of the confirmation to the user.

Furthermore, embodiments also relate to a computer program product which has a program code stored on it that causes the proposed diagnostic system to execute this method, if the program code is run on a computer device. The computer program product may comprise several devices, wherein each device comprises at least a part of the program code. Among these devices may in particular be the mobile device and/or the portable and attachable device as described above.

Advantageous aspects of the various embodiments can be summarized as follows: The aim of this invention is to provide smart, rapid and inexpensive medical diagnosis, in particular veterinarian animal diagnosis. It aims further to develop and/or incorporate a database, or digital library, of illnesses and associated symptoms of the animal or human. It aims further to create an electronic platform, where registered medical experts from all around the world will act as reviewers and consultants. For this aspect, parts of the system - in particular, the data processing unit and the medical diagnosis database - may be hosted in a cloud computing environment, to which the interface provides the input data, and to which the user and the one or more medical experts may connect in order to send and retrieve data. This realization by means of a cloud computing environment is particularly advantageous for connecting the user, who may be near the potentially ill animal or human but without access to a medical professional, remotely with medical experts. The presented system aims to create benchmarks defining the health status of the animal or human, by specifying, measuring and registering the minimum and maximum bounds of health vital signs of the individual. It aims further to use the said benchmarks in performing comparative analysis between the measured parameters and the healthy vital signs benchmark of the animal. It aims further to compile the set of measured vital signs into a preliminary diagnosis, or status report, and to compare the said preliminary diagnosis or report with various illnesses registered in the digital library. Finally, it aims to provide the diagnosis via the electronic platform to one or more medical experts for confirmation, i.e. for review and endorsement, if the diagnosis report is conclusive. If the report is inconclusive, the said platform may refer the preliminary diagnosis to the registered medical expert located most closely to the diagnosed animal or human for further consultation.

One important embodiment of the proposed diagnostic system is configured to deploy all sensor measurements via a smartphone, with proper attachments. Depending on requirements, this solution may be integrated with another smartphone application that caters e. g. to distributed veterinary services, thereby reducing significantly the cost of providing early diagnosis and intervention. The proposed diagnostic system can be of significant importance to any people who raise or otherwise deal with animals.

Smart communication, telemetry, smart imaging, artificial intelligence and contactless sensory applications, properly integrated according to the proposed diagnostic system, can enable pet or other animal owners to achieve a diagnosis a lot faster and a lot cheaper than by calling a veterinarian. One advantage of the proposed diagnostic system over existing solutions is that it simplifies and integrates key diagnostic components in one package, which results in the deployment of remote diagnosis, and provides for early and inexpensive intervention. It enables e. g. a cattle grower, or an owner of animals, to simplify diagnosis and intervention, and to cut costs while providing early and preventive intervention.

### BRIEF DESCRIPTION OF THE DRAWINGS

Various embodiments of the present invention will be described in the following by way of examples only, and with respect to the accompanying drawings, in which:
- Fig. 1: depicts an embodiment of a cloud-based diagnostic system for providing an assessment of a health status of an animal to a user,
- Fig. 2: illustrates a process of providing an assessment of a health status of an animal to a user by means of an embodiment of the presented diagnostic system,
- Fig. 3: depicts an embodiment of the cloud-based diagnostic system for an animal in a shed,
- Fig. 4: depicts an embodiment of the cloud-based diagnostic system for human infant,
- Fig. 5: depicts an embodiment of the cloud-based diagnostic system for human adult,
- Fig. 6: shows steps of a method for providing an assessment of a health status of an animal to a user.

### DETAILED DESCRIPTION

Various examples will now be described more fully with reference to the accompanying drawings in which some examples are illustrated.

Accordingly, while examples are capable of various modifications and alternative forms, the illustrative examples in the figures and will herein be described in detail. It should be understood, however, that there is no intent to limit examples to the particular forms disclosed, but on the contrary, examples are to cover all modifications, equivalents, and alternatives falling within the scope of the disclosure. Like numbers refer to like or similar elements throughout the description of the figures.

The terminology used herein is for the purpose of describing illustrative examples only and is not intended to be limiting. As used herein, the singular forms "a," "an" and "the" are intended to include the plural forms as well, unless the context clearly indicates otherwise. It will be further understood that the terms "comprises," "comprising," "includes" and/or "including," when used herein, specify the presence of stated features, steps, operations, elements and/or components, but do not preclude the presence or addition of one or more other features, steps, operations, elements, components and/or groups thereof.

Unless otherwise defined, all terms (including technical and scientific terms) used herein have the same meaning as commonly understood by one of ordinary skill in the art to which examples belong. It will be further understood that terms, e.g., those defined in commonly used dictionaries, should be interpreted as having a meaning that is consistent with their meaning in the context of the relevant art and will not be interpreted in an idealized or overly formal sense unless expressly so defined herein.

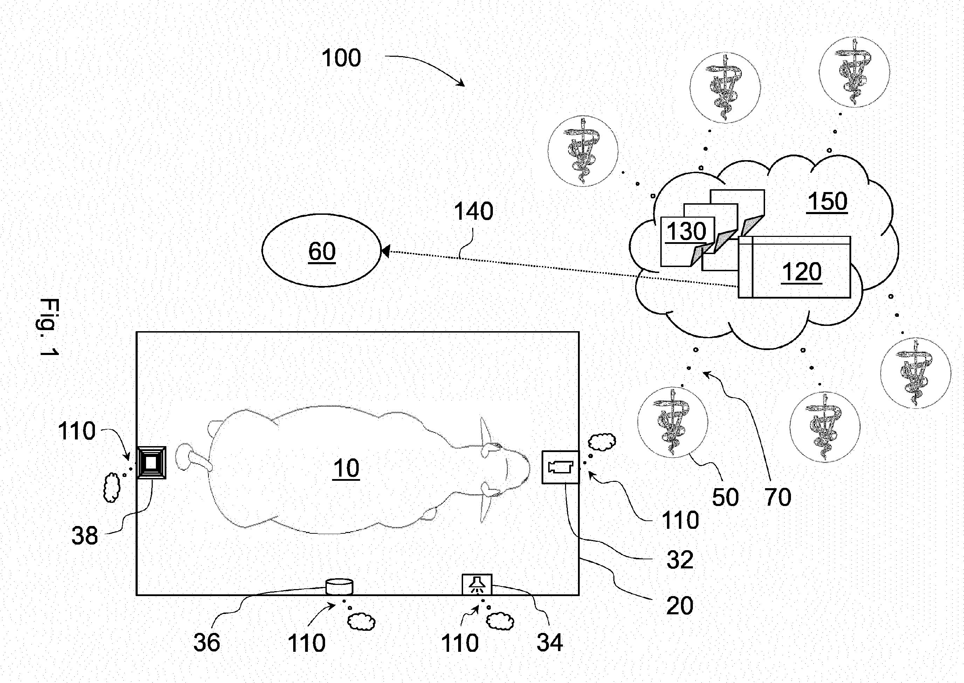

**Fig. 1** depicts an embodiment of a cloud-based diagnostic system 100 for providing an assessment of a health status of an animal 10 to a user 60 according to the present invention. The assessment is based on input data associated with a health status of the animal 10, and on a confirmation 70 of one or more medical experts 50. The diagnostic system comprises an interface 110, configured to collect the input data from several telemetric sensors: a video camera 32, an audio detector 34, a temperature sensor 36, and an odor sensor 38. These sensors 32, 34, 36, 38 are installed within a shed 20 for the animal 10.

The input data is transmitted via the interface 110 to a data processing unit 120, located remotely from the animal 10 and embodied within a cloud computing environment 150. The data processing unit 120 is configured to perform a preliminary diagnosis, based on the input data, of a potential illness of the animal 10. The preliminary diagnosis may be based on recognizing patterns in an outer appearance, movements, and sounds of the animal 10, and on a temperature as well as on an odor in the shed 20, wherein the odor may be connected to feces and/or sweat of the animal 10. In order to arrive at the preliminary diagnosis, the patterns, temperature and odor data are compared with benchmarks or thresholds derived from earlier input data pertaining to the same animal 10, and also with predefined benchmarks or thresholds pertaining to animals of the same kind. The preliminary diagnosis may in particular be based on an automatic recognition of facial features of the animal 10. The preliminary diagnosis may also be based on a rate of change of the input data.

The data processing module 120 is configured to have access to a medical diagnosis database 130, comprising e. g. a digital library of potential illnesses and associated symptoms of the animal 10. The medical diagnosis database 130 holds data adapted to identify a potential illness of the animal 10. According to the present embodiment, the medical diagnosis database 130 is part of the cloud computing environment 150, and thus part of the diagnostic system 100. However, according to further embodiments, the data processing unit 120 may also merely be configured to access a medical diagnosis database 130, which itself is e. g. hosted by a medical organization and thus not part of the diagnostic system 100. In the embodiment depicted in the present figure, the data processing unit 120 is configured to receive the data from the integrated medical diagnosis database 130, and to perform the preliminary diagnosis based on the data from the medical diagnosis database 130.

Signs of illnesses e. g. for cats and dogs, which the data processing unit 120 may be configured to detect, may for example include vomiting or diarrhea, lack of appetite or decreased activity, urinating more or less frequently, coughing, hair loss or itchy skin, stiffness, lameness, or difficulty with rising. Symptoms of a sick animal 10 can further be detected by measuring its temperature, comparing how it looks when it is sick with how it looks when it is healthy, and by detecting odors that are pungent or uncharacteristic.

The data processing unit 120 may in particular be configured to perform a facial recognition of the animal 10. Facial recognition is a critical enabler to simplify animal diagnosis. However, pain itself may not reveal what is ailing the animal 10; other measurements, as e. g. those described before, may be required to aid diagnostics. Facial recognition may be employed to determine if the animal 10 is in pain e.g. due to contortion, which can indicate an ailment of some kind. Deploying facial recognition in this sense aims at recognizing the facial profile of the anima 10, and comparing it to the standard profile. The outcome of the comparison may yield a clear change in the profile indicating pain, and may point in a certain direction if, for example, color changes take place, as e. g. on the sclera, the tongue, or at another position in the face of the animal 10. The contactless audio sensor 32, temperature sensor 36 and odor sensor 38 may complement facial recognition and provide an integrated diagnosis that, when benchmarked with symptoms of various sicknesses of the animal 10, would yield a preliminary diagnosis with varying degrees of confidence.

In a simple embodiment, the diagnostic system 100 may in particular comprise facial recognition software, and additionally require data from contactless temperature measurement, e. g. by a commercially available temperature sensor 36, and data from an odor sensor 38. Together, these measurements, properly integrated, can detect ailments of the animal 10, for which the data processing unit 120 can recommend interventions. This will have significant savings for people who grow cattle in small quantities, such as the cattle farmers in East Africa, but can also be of significant importance to people who raise other valuable animals, such as horses or dogs. The three simple measurements just described, when combined, yield very valuable data on the health of the animal 10, and may actually serve as a very early diagnostic tool thereby enabling early intervention that will both increase the chances of wellbeing of the animal 10 and decrease veterinarian costs.

The data processing unit 120 is further configured to provide the preliminary diagnosis to one or more medical experts 50 in order to obtain the confirmation 70. The data processing unit 120 maybe configured to provide access to the one or more medical experts 50 only if the preliminary diagnosis points at an illness of the animal 10. The data processing unit 120 maybe configured to determine a likelihood value for a disease, injury or other adverse medical condition of the animal 10, and base a decision to inform and/or grant access to the one or more medical experts 50 on this likelihood value. The one or more medical experts 50 may be granted access to the cloud computing environment 150 in order to review the preliminary diagnosis. The confirmation 70 may comprise an endorsement or validation of the preliminary diagnosis, but also indicate a wrong diagnosis, a necessity for further tests, or any other opinion of any of the one or more medical experts 50.

The data processing unit 120 is further configured to provide a result 140 of the confirmation 70 to the user 60. According to the embodiment depicted in this figure, the user 60 may e. g. be an owner, a keeper, or a herdsman, or a caretaker of the animal 10. The result 140 of the confirmation 70 may comprise elements of the confirmation. In particular, it may comprise the endorsement of the preliminary diagnosis by any of the one or more medical experts 50, thus corroborating the preliminary diagnosis. It may also indicate a wrong diagnosis, a necessity for further tests, or any other opinion of one of the medical experts 50. The result 140 of the confirmation 70 may also comprise additional advice for treating the animal 10, from the one or more medical experts 50 and/or added automatically by the processing unit 120.

The data processing unit 120 may be configured to notify the user 60 that a result 140 of the confirmation 70 is available. The provision of the result 140 may be implemented by means of a remote access to the cloud computing environment 150 in order to retrieve the result 140. The data processing unit 120 may also be configured to send the result 140 to the user 60 via electronic mail or text message.

The data processing unit 120 may be configured to improve, by adaptive learning or machine learning, the process for providing the preliminary diagnosis. To this end, the data processing unit 120 may comprise an artificial intelligence module, configured to improve a neural network through machine learning. An input for machine learning may include input data, data from the medical diagnosis database 130, confirmations 70, and results 140 from the same animal or human, or from a different animal or human. The data processing unit 120 may therefore in particular be adapted to improve the process for providing the preliminary diagnosis based on the expertise of the medical experts 50.

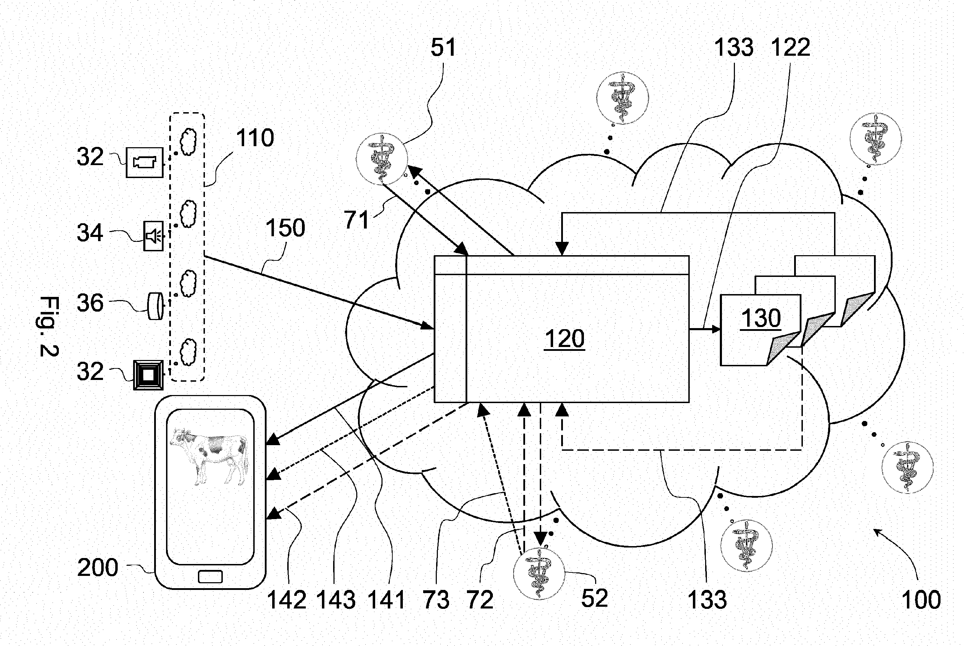

**Fig. 2** illustrates a process of providing an assessment of a health status of an animal 10 to a user 60 by means of an embodiment of the presented diagnostic system 100. In the upper left part of the figure, sensors 32, 34, 36, 38 are depicted which provide input data associated with the health status of the animal 10. The input data is collected by the interface 110 and conveyed via a data connection 115. The data connection 115 may in particular comprise a wireless transmission, e.g. via a mobile network or a WWAN, to the data processing unit 120. The sensor data may be conveyed individually, or after an aggregation performed by the interface 110. The data processing unit 120 is configured to process the input data and send a signal 122 to the medical diagnosis database 130 (or digital library). In embodiments where the medical diagnosis database 130 is a clinical decision support system (CDSS), the signal 122 may comprise a result of a data preparation process applied to the input data, with the result adapted to serve as input for the CDSS. In other embodiments, the signal 122 may comprise a list suited for a diagnosis by pattern recognition, or matching of data.

The figure depicts two exemplary conclusions 133, 135 from processing the signal 122 through the medical diagnosis database 130: A first conclusion 133 yields, in the data processing unit 120, a preliminary diagnosis in which the health status is deemed or classified as at least reasonably well identified. In particular, the first conclusion 133 may lead to a preliminary diagnosis which points at a particular illness or condition of the animal 10. Depicted is also an exemplary second conclusion 135, which yields a preliminary diagnosis by which the health status is deemed, by the data processing unit 120, as unclassifiable or inconclusive.

In the present embodiment, the data processing unit 120 is configured to provide the preliminary diagnosis based on the findings of the medical diagnosis database 130 (i. e., either on the first conclusion 132, or on the second conclusion 135) to a medical expert 51, 52. In the figure, a first medical expert 51 is depicted as receiving the preliminary diagnosis in the example where it clearly points to a particular illness. The first medical expert 51 returns a first confirmation 71, corroborating the preliminary diagnosis. A second medical expert 52 is depicted as receiving the diagnosis in the example where it indicates an unclear health status. The second medical expert 52 may return, again by way of example, a second confirmation 72, providing a diagnosis of his own, based on the preliminary diagnosis. Depicted is, as a further example, that the second medical expert 52 may send a third confirmation 73 which corroborates the fact that the input data yields an inconclusive information about the health status of the animal 10.

Once the data processing unit 120 has received one of the exemplary confirmations 71, 72, 73, it is configured to correspondingly provide a result 141, 142, 143 of the respective confirmation 71, 72, 73 to the user 60. In the present embodiment, the data processing unit 120 is configured to send information to a mobile device 200. In the example which lead to the first confirmation 71 (which corroborates the preliminary diagnosis pointing to a particular illness), the data processing unit 120 may send a result 141 in form of a report to be presented in a software application on the mobile device 200. The report may include additional statements by the first medical expert 51, or instructions automatically added by the data processing unit 120, pertaining to a standard procedure to cure the illness. In the example where the second medical expert 52 has presented his own diagnosis based on the previously inconclusive preliminary diagnosis, a corresponding result 142 may take the form of a report similar to the one just described, containing the diagnosis of the second medical expert 52. In the case where the second medical expert 52 has opined that the input data yields an inconclusive information about the health status of the animal 10, the data processing unit 120 may send, as a result 143, this confirmation together with possible further instructions by the second medical expert 52. In this example, the data processing unit 120 may also provide the user 60 with contact details of a medical expert or veterinarian located in a proximity of a position of the animal 10.

In embodiments of the diagnostic system 100, the data processing unit 120 may be configured to aggregate responses by several different medical experts 50, 51, 50, and provide an according result 140 to the user 60.

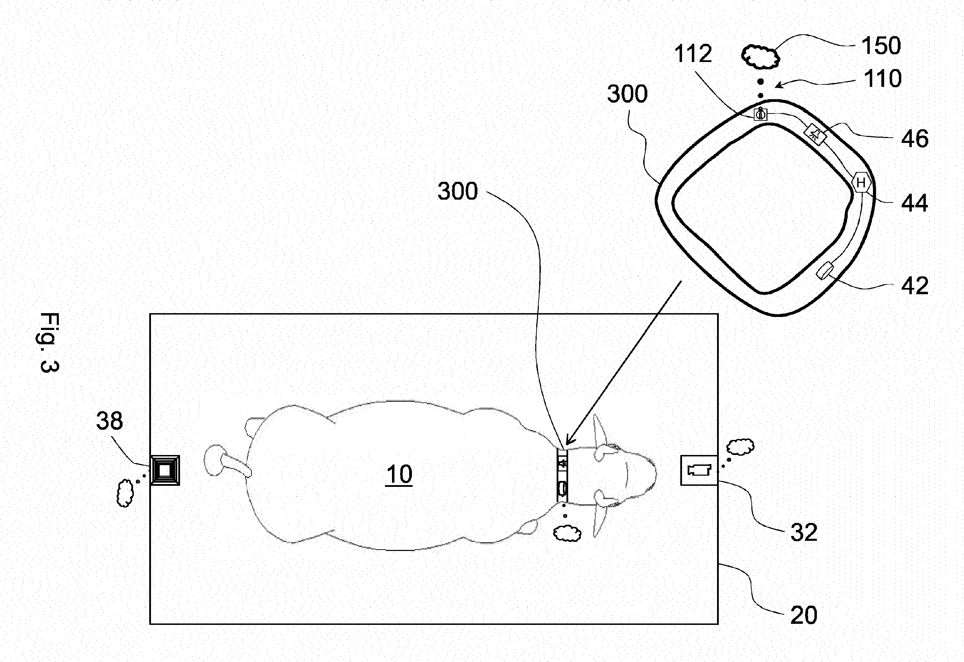

**Fig. 3** depicts an animal 10 in a shed 20, together with a video sensor 32 and an odor sensor 38, connected to the cloud computing environment 150 by the interface 110 of an embodiment of the diagnostic system 100. The animal 10 wears a collar 300 comprising several contact-based sensors 42, 44, 46. The collar 300 is also represented separately in the upper right of the figure. In the present embodiment, the collar 300 comprises a temperature sensor or thermometer 42, configured to measure a body temperature of the animal 10. It also comprises a heart rate or pulse sensor 44, configured to measure a heart rate or pulse of the animal 10. The collar 300 further comprises a microphone 46, configured to detect breathing sounds from the animal 10. The collar 300 also includes a communication chip 112 as a part of the interface 110, which is configured to collect sensor data from the sensors 42, 44, 46 and to send the sensor data as input data to the data processing unit 120.

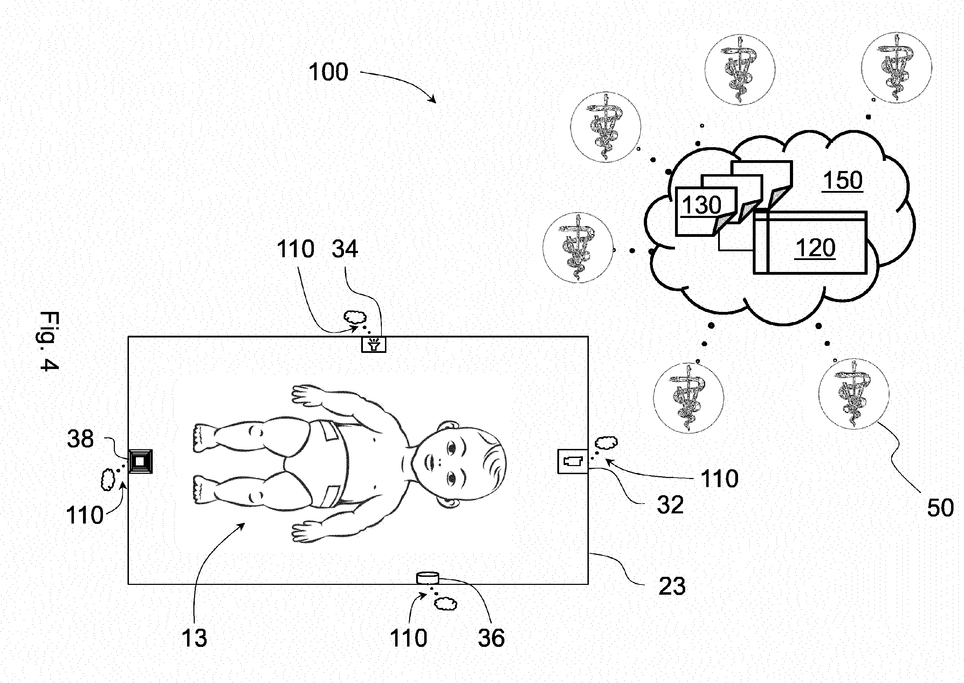

**Fig. 4** depicts an embodiment of the diagnostic system 100 adapted to provide an assessment of a health status of a human infant 13. The infant 13 is depicted in a cradle 23, which comprises a frame holding a video sensor 32, a microphone 34, a temperature sensor 36, and an odor sensor 38, connected to the data processing unit 120 by the interface 110 of the diagnostic system 100. The diagnostic system 100 is adapted to provide an assessment of the health status of the infant 13. The data processing unit 120 does not require conscious answers from the infant 13 as input data.

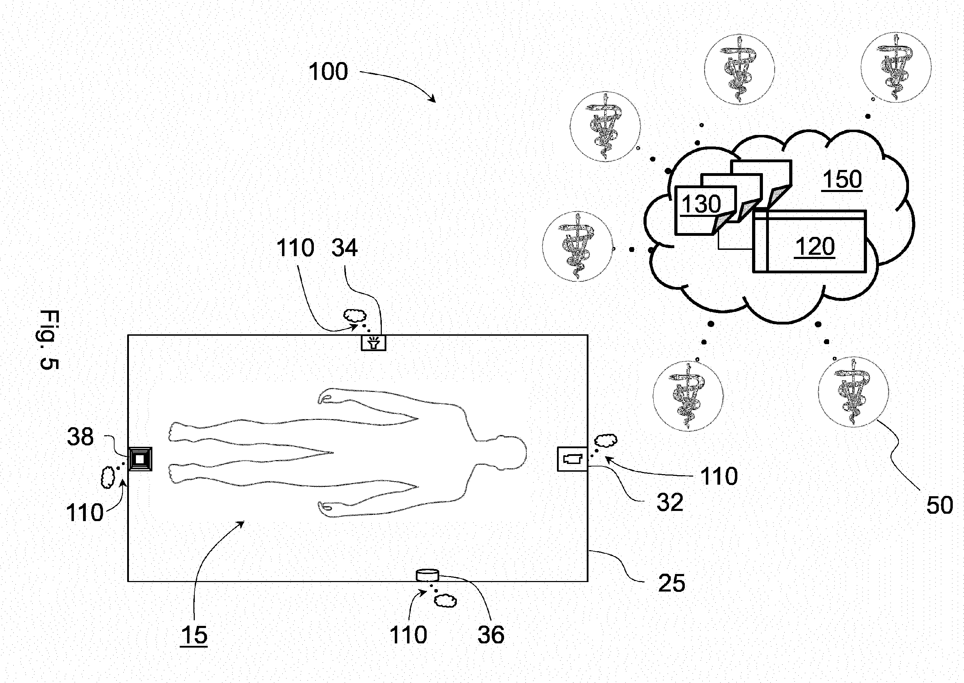

**Fig. 5** depicts an embodiment of the diagnostic system 100 adapted to provide an assessment of a health status of a human adult 15. The adult 15 is depicted in a bed 25, which comprises a frame adapted to hold a video sensor 32, a microphone 34, a temperature sensor 36, and an odor sensor 38, connected to the data processing unit 120 by the interface 110 of the diagnostic system 100. The diagnostic system 100 is adapted to provide an assessment of the health status of the adult 15. Advantageously, the data processing unit 120 does not require conscious answers from the adult 15 as input data. This embodiment of the diagnostic system 100 is beneficial for humans who have difficulty communicating, e. g. due to a disability, or to language problems.

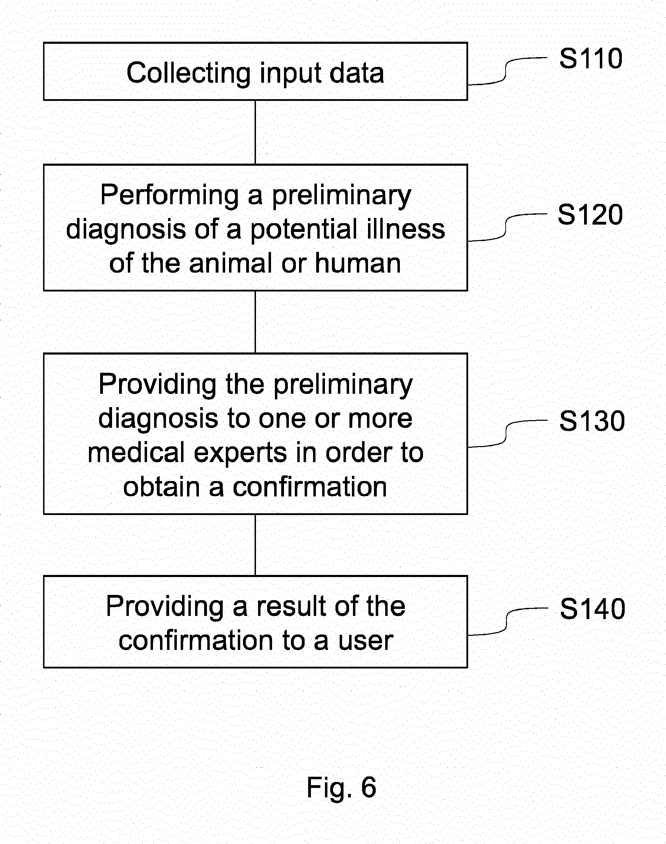

**Fig. 6** shows steps of a method for providing an assessment of a health status of an animal 10 or human 13, 15 to a user 60. One step of the method comprises collecting (S110) input data from a variety of sensors 32, 34, 36, 38, 42, 44, 46, around the animal 10 or human 13, 15. A further step of the method comprises performing (S120) a preliminary diagnosis, based on the input data, of a potential illness of the animal 10 or human 13, 15. Another step of the method comprises providing (S130) the preliminary diagnosis to one or more medical experts 50 in order to obtain the confirmation 70. A further step of the method comprises providing (S140) a result 140 of the confirmation to the user 60.

This method may also be a computer-implemented method. A person of skill in the art will readily recognize that steps of the above-described method may be performed by programmed computers. Embodiments are also intended to cover program storage devices, e.g., digital data storage media, which are machine or computer readable and encode machine-executable or computer-executable programs of instructions, wherein the instructions perform some or all of the acts of the above-described method, when executed on the computer or processor.

The description and drawings merely illustrate the principles of the disclosure. It will thus be appreciated that those skilled in the art will be able to devise various arrangements that, although not explicitly described or shown herein, embody the principles of the disclosure and are included within its scope.

Furthermore, while each embodiment may stand on its own as a separate example, it is to be noted that in other embodiments the defined features can be combined differently, i.e. a particular feature described in one embodiment may also be realized in other embodiments. Such combinations are covered by the disclosure herein, unless it is stated that a specific combination is not intended.

### List of reference signs

- 10: animal
- 13: human infant
- 15: human adult
- 20: shed
- 23: cradle
- 25: bed
- 32: video sensor or camera
- 34: audio sensor or microphone
- 36: telemetric temperature sensor or thermometer
- 38: odor sensor
- 42: thermometer
- 44: heart rate or pulse sensor
- 46: contact-based audio sensor or microphone
- 50, 51, 52: medical experts
- 60: user
- 70, 71, 72, 73: confirmations
- 100: diagnostic system
- 110: interface
- 112: communication chip
- 120: data processing unit
- 122: signal
- 130: medical diagnosis database
- 133, 135: conclusions
- 140, 141, 142, 143: results
- 150: cloud computation environment
- 200: mobile device, e. g. smartphone, laptop, or tablet
- 300: portable and attachable device, e. g. collar, harness, or cuff

## Claims

1. A cloud-based diagnostic system (100) for providing an assessment of a health status of an animal (10) or human (13, 15) to a user (60), the diagnostic system (100) comprising
an interface (110), configured to collect input data associated with a health status of the animal (10) or human (13, 15); and
a data processing unit (120), configured
to perform a preliminary diagnosis, based on the input data, of a potential illness of the animal (10) or human (13, 15),
to provide the preliminary diagnosis to one or more medical experts (50, 51, 52) for obtaining a confirmation (70, 71, 72, 73), and
to provide a result (140, 141, 142, 143) of the confirmation (70, 71, 72, 73) to the user (60).

2. The diagnostic system (100) of claim 1, wherein the processing module (120) is configured to process, as input data, sensor data from one or more of the following telemetric sensors:
a visual camera (32),
an audio detector (34),
a temperature sensor (36),
an odor sensor (38).

3. The diagnostic system (100) of any of the preceding claims, wherein processing unit (120) is further configured to process, as input data, sensor data of one or more of the following contact-based sensors:
a sensor for detecting body temperature (42),
a heartbeat or pulse sensor (44),
a sensor for detecting respiratory sounds (46),
a sensor for measuring blood pressure.

4. The diagnostic system (100) of any of the preceding claims, wherein the data processing unit (120) is configured to keep a record of the input data, and to provide the preliminary diagnosis based on one or more of the following:
benchmarks or thresholds derived from input data,
predefined benchmarks or thresholds to be compared to the input data,
a rate of change of the input data,
an automatic recognition of facial features of the animal (10) or human (13, 15).

5. The diagnostic system (100) of any of the preceding claims, further comprising a medical diagnosis database (130) for storing data adapted to identify a potential illness, wherein the data processing unit (120) includes an artificial intelligence module, and is configured to receive the data from the medical diagnosis database (130) and to perform the preliminary diagnosis based on the data from the medical diagnosis database (130) with support of the artificial intelligence module.

6. The diagnostic system (100) of claim 5, wherein the data processing unit (120) is configured to keep a record of preliminary diagnoses, confirmations (70, 71, 72, 73), and/or results (140, 141, 142, 143), and to carry out, by means of the artificial intelligence module, a learning algorithm, and to perform the preliminary diagnosis based on the record and on the learning algorithm.

7. The diagnostic system (100) of any of the preceding claims, wherein the data processing unit (120) is configured to establish a communication channel between the user (60) and at least one of the one or more medical experts (50, 51, 52).

8. The diagnostic system (100) of any of the preceding claims, wherein the processing unit (120) is configured to process, as input data, output data from a mobile test device providing test results associated with body fluids such as blood, urine, saliva or body excrements such as dung or vomit.

9. A mobile device (200), configured to connect to the diagnostic system (100) of any of the preceding claims, and to receive the result (140, 141, 142, 143) of the confirmation (70, 71, 72, 73).

10. A portable device (300) attachable to the animal (10) or human (13, 15), the portable device (300) comprising one or more sensors (42, 44, 46) configured to produce sensor data, and further configured to send the sensor data to the interface (110) of the diagnostic system (100) of any of the claims 1 to 9.

11. A method for providing an assessment of a health status of an animal (10) or human (13, 15) to a user (60), the method comprising
collecting (S110) input data associated with a health status of the animal (10) or human (13, 15);
performing (S120) a preliminary diagnosis, based on the input data, of a potential illness of the animal (10) or human (13, 15),
providing (S130) the preliminary diagnosis to one or more medical experts (50, 51, 52) in order to obtain the confirmation (70, 71, 72, 73); and
providing (S140) a result (140, 141, 142, 143) of the confirmation (70, 71, 72, 73) to the user (60).

12. A computer program product, having stored a program code for controlling the diagnostic system of any of the claims 1 to 8 to perform the method of claim 11 if the program code is executed on a computer or data processing unit.
